(19) Europäisches Patentamt
European Patent Office
Office européen des brevets

(11) Publication number : **0 326 326 B1**

(12) # EUROPEAN PATENT SPECIFICATION

(45) Date of publication of patent specification : **07.01.93 Bulletin 93/01**

(51) Int. Cl.$^5$ : **C07C 323/00,** A61K 31/195

(21) Application number : **89300651.0**

(22) Date of filing : **24.01.89**

(54) **Cysteine derivatives.**

(30) Priority : **25.01.88 JP 14189/88**

(43) Date of publication of application : **02.08.89 Bulletin 89/31**

(45) Publication of the grant of the patent : **07.01.93 Bulletin 93/01**

(84) Designated Contracting States : **AT BE CH DE FR GB IT LI LU NL SE**

(56) References cited :
**GB-A- 1 530 071**
**GB-A- 2 096 145**
**US-A- 4 241 086**
**US-A- 4 255 446**
**US-A- 4 305 958**

(73) Proprietor : **SANTEN PHARMACEUTICAL CO., LTD.**
**9-19, 3-Chome, Shimoshinjo**
**Higashiyodogawa-ku Osaka533 (JP)**

(72) Inventor : **Morita, Takakazu 302**
**Kitasakurazuka Park Heim**
**6-8 Kitasakurazuka 3-chome**
**Toyonaka-shi Osaka (JP)**
Inventor : **Mita, Shiro**
**26-304 Higashiyamacho 7-chome**
**Ashiya-shi Hyogo (JP)**
Inventor : **Iso, Tadashi**
**22-18 Kiyomidai 1-chome**
**Kawachinagano-shi Osaka (JP)**
Inventor : **Kawashima, Yoichi**
**8-54 Oharanonishisakaidanicho 3-chome**
**Nishikyoku**
**Kyoto-shi Kyoto (JP)**

(74) Representative : **Pearce, Anthony Richmond et al**
**MARKS & CLERK Alpha Tower Suffolk Street**
**Queensway Birmingham B1 1TT (GB)**

EP 0 326 326 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

Detailed Description of the Invention

This invention relates to cysteine derivatives having pharmacological activity and to a process of preparation of such compounds.

GB-A-2096145 discloses intra- or intermolecular disulfides of the formula:-

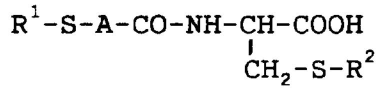

wherein $R^1$ and $R^2$ each represent a hydrogen atom, a lower alkyl group, an aryl group, an aralkyl group, a lower alkanoyl group or an aroyl group, and A represents a straight or branched chain $C_1$-$C_3$ alkylene group. The use of such compounds for the treatment of liver damage, cataracts and immuno-mediated diseases is disclosed. N,N-Bis [2-methyl-2-(methylthio)propanoyl]-L-cystine, N,N′-[2,2′-dithiobis(2-methylpropanoyl)]bis(S-methyl-L-cysteine),and (4R)-7,7-dimethyl-6-oxo-3,4,6,7-tetrahydro-1,2,5-dithiazepine-4-carboxylic acid are specifically disclosed.

US-A-4255446 discloses compounds of the formula:-

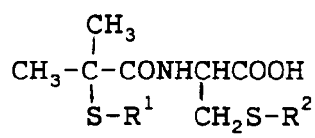

wherein $R^1$ and $R^2$ are lower alkanoyl, benzoyl, lower alkyl or lower alkyl substituted by carboxy, and the use thereof for suppressing liver disorders.

US-A-4305958 and GB-A-1530071 disclose compounds of the formula:-

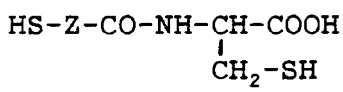

wherein Z is selected from $CH_3$-CH <,$(CH_3)$C <, $(CH_2)_2$ < and $CH_2$ <, acting as agents for liquefaction of sputum.

US-A-4241086 discloses the use of N-(2 mercapto-2-methylpropanoyl)-L-cysteine for the treatment of rheumatism and arthritis.

According to the present invention, there is provided a compound of the formula [I]

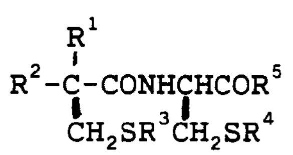

[I]

wherein

$R^1$ and $R^2$ are the same lower alkyl;

$R^3$ and $R^4$ are the same or different and are selected from hydrogen, lower alkyl, lower alkanoyl, optionally substituted phenyl lower alkyl and optionally substituted phenylcarbonyl, wherein the optional substituents are lower alkyl, lower alkoxy or halogen; and

$R^5$ is hydroxy, lower alkoxy, amino or lower alkylamino; or a pharmaceutically acceptable salt thereof.

The terms defined above are explained as follows in more detail.

The term "lower alkyl" intends to designate straight or branched $C_1$ - $C_6$ lower alkyl exemplified by methyl, ethyl, propyl, isopropyl and hexyl.

The term "lower alkanoyl" intends to designate straight or branched $C_1$ - $C_6$ lower alkanoyl exemplified by acetyl, propionyl, pivaloyl and hexanoyl.

There are various studies on cysteine derivatives and such studies were reported in US-A-4305958, US-A-4241086, US-A-4255446 etc.

The cysteine derivatives are known to have many kinds of efficacy such as suppression of liver disorders and anti-rheumatism. But there are very few studies which report the influence on pharmacological efficacy by incorporation of alkylene group in the side chain of cysteine derivatives, expansion of the alkylene length of the side chain or by substitution of radicals. So we studied cysteine derivatives in more detail.

We synthesized various novel cysteine derivatives and examined their pharmacological effects, especially the effect of incorporation of alkylene group in the side chain of the cysteine derivatives and expansion of the alkylene length of the side chain.

From the results of the pharmacological examination, which are described later in the pharmacological test, we found that the compounds of this invention have an excellent supressing effect on liver disorders and immunomodulating effect.

The compounds of the formula[I] can be prepared by the similar methods to those described in US-A-4305958 and U-SA-4255446 or Japanese Patent Publication 12119/1984.

The typical methods are shown below.

(A)

The compound of the formula[I] can be prepared by the reaction of an amino acid derivative of the formula[II] with a carboxylic acid derivative of the formula[III] or an active derivative thereof.

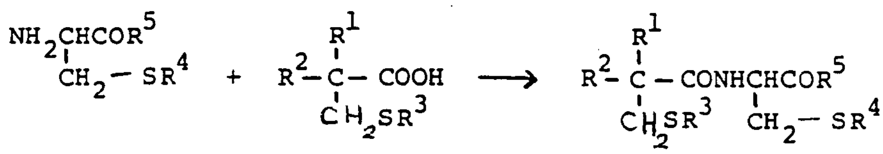

[II] [III] [I]

The active derivative referred to above is a reactive derivative of the carboxylic acid exemplified by the acid chloride, the acid anhydride and the mixed acid anhydride. The active derivative of the compound the formula[III] can be converted into the compound of the formula[I] by the usual method such as Schotten-Baumann method which is generally used for condensation of amine derivatives with carboxylic acid derivatives.

The carboxylic acid of the formula[III] can be converted directly into the compound of the formula[I] using a condensing agent such as N,N'-dicyclohexylcarbodiimide(DCC).

It is not necessary to specify a reaction condition such as temperature or reaction time.

(B)

The compound of the formula[I] can be prepared by the reaction of the amino acid derivative of the formula[II] with a polythioester of the formula[IV],

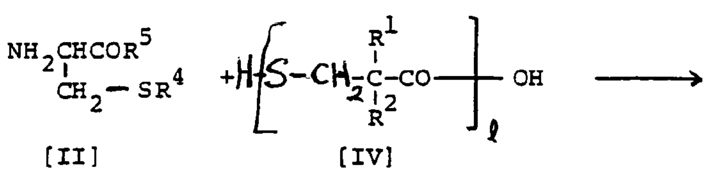

[I]

[II] [IV]

wherein $\ell$ is a polymerization degree having a mean molecule weight of about 200-1500.

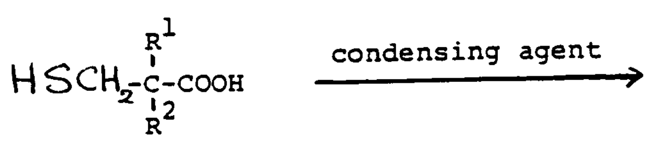


[IV]

[V]

The compound of the formula[Iv] can be prepared from the corresponding monomer of the formula[V] using a condensing agent such as DCC in an organic solvent.

It is not necessary to specify conditions of condensation of the compound of the formula[II] with the compound of the formula[IV], but, the reaction is usually performed in a presence of base such as sodium carbonate or potassium carbonate.

In connection with the above methods (A) and (B):

1) When $R^3$ and/or $R^4$ are/is hydrogen, if necessary, the group(s) can be converted into other groups than hydrogen after the above-mentioned reaction. The conversion can be performed using a known method which is used for an introduction of a protective group etc. to a thiol group.

2) When $R^5$ is hydroxy, if necessary, the group can be converted into ester or amide after the above-mentioned reaction. The conversion can be performed using a known method which is used for conversion of a carboxylic group to ester or amide.

3) When the group defined in $R^3$ , $R^4$ or $R^5$ is used as protective group, if necessary, such group can be removed after the above-mentioned reaction. The removal can be performed by a known method.

The compound of the formula[I] can be converted into pharmaceutically acceptable salts using an inorganic or an organic base.

Examples of the salts are sodium salt, potassium salt, calcium salt, magnesium salt, ammonium salt, diethylamine salt and triethanolamine salt.

The compounds of this invention have stereoisomers because of the existence of one or more asymmetric carbon atoms and these isomers are included in this invention.

A liver disorder model caused by an administration of $CCl_4$ to a rat is widely used to examine efficacy of a compound on liver diseases.

GOT and GPT values in the serum are used as an indication of a degree of liver disorder. If the value, which is raised by liver disorder, falls by an administration of a compound, the compound is judged effective on liver disorder.

As the result of the experiment, whose detailed data are shown in the pharmacological test, using the compounds of this invention, we found that the activity of serum transaminase in the group treated with the compound of this invention is significantly decreased as compared with that in the untreated group. The experiment proves that the compounds of this invention have a suppressive effect on liver disorder.

Recently, immunity has been thought to be closely related to the mechanism of development and chronicity of liver disorder. To examine the influence of the compound of this invention of the immune system, we examined the immune response against sheep red blood cells in mice, which is usually used to examine immunomodulating effect.

This experimental method is to examine the efficacy on the immune system according to increase or decrease of the number of haemolytic plaque-forming cells of mouse spleen cells. As shown in the pharmacological test, the compound of this invention shows an excellent immunosuppressive effect.

A compound, which has a similar chemical structure to the compounds of this invention, is disclosed in above-mentioned US-A-4305958. It is generally recognized that very slight modification of the chemical structure greatly influences the efficacy of a compound. So, we examined how the modification of the chemical structure influences to the efficacy.

We made the comparative test on the immunosuppressive effect of the compound of this invention and known compound represented by the formula[VI].

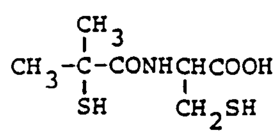

[VI]

As shown in the pharmacological test, the compound of this invention shows more effect than the compound described in US-A-4305958.

As the result, we found that the compound of this invention must be a new type of drug for liver diseases because the compound decreased the value of GOT and GPT in serum and suppressed the immunity.

Furthermore, the compound of this invention can be used as a drug for autoimmune diseases such as rheumatoid arthritis.

The compound(s) of this invention can be administered either orally or parenterally. Examples of dosage forms are tablet, capsule, powder, granule, suppository, injection, eye drops and percutaneous.

The dosage is adjusted depending on symptom, dosage form, etc., but usual daily dosage is 1 to 5000mg in one or a few divided doses.

Examples of preparations of the compounds and formulations are shown below.

## EXAMPLES

### Reference Example

N-(2-Mercapto-2-methylpropionyl)-DL-homocysteine (compound No.1)

To a stirred solution of 2-mercapto-2-methylpropionic acid (40.4g) in ethyl acetate(200ml), N,N′-dicyclohexylcarbodiimide (69.3g) dissolved in ethyl acetate(200ml) was added dropwise under ice-cooling. After the addition, the reaction mixture was stirred for 1 hour at room temperature and filtered. The filtrate was concentrated in vacuo. To the residue, 400ml of N,N-dimethylformamide(DMF) was added to give DMF solution of polythioester.

To a mixture of DL-homocysteine(37.9g), potassium carbonate(77.4g), water(400ml) and DMF(100ml), the polythioester solution was added and the reaction mixture was stirred overnight at room temperature. Water(21) was added to the reaction mixture and washed with ethyl acetate. The aqueous layer was acidified with 6 N hydrochloric acid and extracted with ethyl acetate. The organic layer was washed with saturated sodium chloride solution, dried over anhydrous sodium sulfate and concentrated in vacuo. The oily residue was purified by a silica gel column chromatography and recrystallized with isopropyl ether to give 39.8g(59.9%) of the titled compound.

mp 102 - 104°C (isopropyl ether)

IR(KBr, $cm^{-1}$) 3405, 3335, 2536, 1714, 1599, 1524, 1412, 1296, 1270, 1254, 1213, 1186, 658

### Example 1

N-(2-2-Dimethyl-3-mercaptopropionyl)-L-cysteine (compound No.2)

DMF solution of polythioester was prepared using 3-mercaptopivalic acid(40.3g) and N,N′-dicyclohexylcarbodiimide (61.9g) by a similar method to that described in the Reference Example Example.

The polythioester solution was added to a mixture of L-cysteine hydrochloride monohydrate(52.7g), potassium carbonate (124g), water (400ml) and DMF(100ml). The mixture was stirred overnight at room temperature and followed by a similar method treatment to that described in the Reference Example to give 47.2g(66.3%) of the titled compound.

mp 83.5 - 85.5°C (isopropyl ether)

IR (KBr, $cm^{-1}$) 3352, 2596, 1728, 1630, 1528, 1424, 1403, 1296, 1204, 870, 593

Optical Rotation $[\alpha]_D^{25}$ -3.3° (c=1.0, methanol) and $[\alpha]_D^{25}$ +57.8° (c=1.0, chloroform)

The following compound was prepared by the similar method as Example 1.

N-[(2-Ethyl-2-mercaptomethyl)butyryl]-L-cysteine (compound No.3)

IR (KBr, $cm^{-1}$) 3350,1726,1628,1526

### Example 2

S-Benzyl-N-(3-benzylthio-2,2-dimethylpropionyl)-L-cysteine (compound No.4)

To a stirred solution of S-benzyl-L-cysteine(11.5g) in 2 N sodium hydroxide solution(68ml), 3-benzylthio-2,2-dimethylpropionyl chloride (14.6g) dissolved in ether(10ml) was added dropwise under ice-cooling. After the addition, the reaction mixture was stirred for 20 minutes under ice-cooling and 2 hours at room temperature. The reaction mixture was acidified with 6 N hydrochloric acid and extracted with ethyl acetate. The organic layer was washed with saturated sodium chloride solution, dried over anhydrous sodium sulfate and concentrated in vacuo. The oily residue was purified by a silica gel column chromatography to give 19.4g(85.1%) of the titled compound.

IR(film, $cm^{-1}$) 3344, 1731, 1620, 1514, 1495, 1236, 1199, 700

Optical Rotation $[\alpha]_D^{25}$ -31.5° (c=1.1, methanol)

The following compound was prepared by the similar method as Example 2.

S-Benzyl-N-(3-benzylthio-2,2-dimethylpropionyl)-D-cysteine (compound No.5)
IR(film, $cm^{-1}$) 3344, 1729, 1620, 1513, 1495, 1236, 1200, 699
Optical Rotation $[\alpha]_D^{25}$ +33.0° (c=1.0, methanol)

Example 3

N-(2,2-Dimethyl-3-mercaptopropionyl)-L-cysteine (compound No.2)

To a solution of S-benzyl-N-(3-benzylthio-2,2-dimethylpropionyl)-L-cysteine(18.0g) in liquid ammonia(250ml), metallic sodium (5.0g) cut into small pieces was added. To this solution, ammonium chloride was added and then liquid ammonia was evaporated. To the residue, water was added, and the solution was washed with ethyl acetate. The aqueous layer was acidified with 6 N hydrochloric acid and extracted with ethyl acetate. The organic layer was washed with saturated sodium chloride solution, dried over anhydrous sodium sulfate and concentrated in vacuo. The oily residue was purified by a silica gel column chromatography and recrystallized with a mixture of ethyl acetate and cyclohexane to give 7.1g(69.6%) of the titled compound. The physical data were identical with those of the compound obtained in Example 1.

The following compound was prepared by the similar method as Example 3.

N-(2,2-Dimethyl-3-mercaptopropionyl)-D-cysteine (compound No.6)
mp 83.5 - 85.0°C (ethyl acetate - cyclohexane)
IR(KBr, $cm^{-1}$) 3352, 2595, 1725, 1624, 1522, 1421, 1401, 1295, 1201, 867, 590
Optical Rotation $[\alpha]_D^{25}$ -57.1° (c=1.0, chloroform)

Example 4

N-[2,2-Dimethyl-3-(methylthio)propionyl]-S-methyl-L-cysteine dicyclohexylamine salt (compound No.7)

N-(2,2-dimethyl-3-mercaptopropionyl)-L-cysteine(23.7g) was dissolved in a solution of potassium carbonate(41.5g) in water (150ml) under ice-cooling. To this solution, methyl iodide (36.9g)was added. After the addition, the reaction mixture was stirred for 30 minutes under ice-cooling and for 1 hour at room temperature. To the reaction mixture, 1 N iodine solution(15ml) was added and the solution was washed with ethyl acetate. The aqueous layer was acidified with 6 N hydrochloric acid and extracted with ethyl acetate. The organic layer was washed with sodium hydrogen sulfide solution and saturated sodium chloride solution, dried over anhydrous sodium sulfate and concentrated in vacuo. The oily residue was purified by a silica gel column chromatography. Dicyclohexylamine was added to the oily product to give crystals. The crystals were recrystallized with a mixture of ethyl acetate and hexane to give 29.0g(65.0%) of the titled compound.
mp 98 - 99.0° (ethyl acetate - hexane)
IR(KBr, $cm^{-1}$) 3380, 2912, 2848, 1635, 1561, 1498, 1409, 1392, 587
Optical Rotation $[\alpha]_D^{25}$ +18.6° (c=1.0, methanol)

Example 5

N-[2,2-Dimethyl-3-(methylthio)propionyl]-L-cysteine dicyclohexylamine salt (compound No.8)

L-Cysteine hydrochloride monohydrate (15.6g) was dissolved in a solution of potassium carbonate(46.8g) in water(150ml). To this solution, 2,2-dimethyl-3-(methylthio)propionyl chloride (15.6g) was added dropwise. After the addition, the reaction mixture was stirred for 1 hour at room temperature and washed with ethyl acetate. The aqueous layer was acidified with 6 N hydrochloric acid and extracted with ethyl acetate. The organic layer was washed with saturated sodium chloride solution, dried over anhydrous sodium sulfate and concentrated in vacuo. The oily residue was purified by a silica gel column chromatography. Dicyclohexylamine was added to the oily product to give crystals. The crystals were recrystallized with a mixture of ethyl acetate and hexane to give 28.5g(70.1%) of the titled compound.
mp 122.0 - 123.5° (ethyl acetate - hexane)
IR(KBr, $cm^{-1}$) 3404, 2920, 2852, 1629, 1560, 1483, 1412, 1383
Optical Rotation $[\alpha]_D^{25}$ +35.0° (c=1.1, methanol)

Example 6

N-(2,2-Dimethyl-3-(benzoylthio)propionyl)-S-methyl-L-cysteine (compound No.9)

To a stirred mixture of S-methyl-L-cysteine(6.5) dissolved in 1.6 M aqueous potassium carbonate solution(50ml) and acetone (50ml), 2,2-dimethyl-3-(benzoylthio)propionyl chloride (bp 138 - 145°/1.OmmHg, 12.3g) was added dropwise under ice-cooling. After 10 minutes, the reaction mixture was stirred for 2 hours at room temperature. The reaction mixture was acidified with 2 N hydrochloric acid and extracted with ethyl acetate. The organic layer was washed with water, dried over anhydrous magnesium sulfate and concentrated in vacuo. The oily residue was purified by a silica gel column chromatography to give 12.4g(72%) of the titled compound.

As dicyclohexylamine salt:

mp 101 - 102°C (ethyl acetate - hexane)

IR(KBr, $cm^{-1}$) 3372, 2912, 2852, 1632

Optical Rotation $[\alpha]_D^{25}$ +11.8° (c=1.0, methanol)

Example 7

N-(2,2-Dimethyl-3-mercaptopropionyl)-S-methyl-L-cysteine (compound No.10)

To a stirred solution of N-[2,2-dimethyl-3-(benzoylthio)-propionyl]-S-methyl-L-cysteine(10.0g) in methanol(20ml), 28% ammonia water(40ml) was added and the mixture was stirred for 2 hours at room temperature. The reaction mixture was washed with ethyl acetate, acidified with 6 N hydrochloric acid and extracted with ethyl acetate.

The organic layer was washed with saturated sodium chloride solution, dried over anhydrous magnesium sulfate and concentrated in vacuo. The oily residue was purified by a silica gel column chromatography to give 5.8g(82%) of the titled compound.

As dicyclohexylamine salt:

mp 97 - 99°C (ethyl acetate - hexane)

IR(KBr, $cm^{-1}$) 3364, 2916, 2852, 1635, 1559

Optical Rotation $[\alpha]_D^{25}$ +9.5° (c=1.05, methanol)

Formulation Example

1) Tablet

| | |
|---|---|
| compound No.2 | 100mg |
| crystalline cellulose | 20mg |
| lactose | 40mg |
| L-hydroxypropylcellulose | 5mg |
| magnesium stearate | 5mg |
| | 170mg |

2) Capsule

| | |
|---|---|
| compound No.2 | 5mg |
| magnesium stearate | 3mg |
| lactose | 142mg |
| | 150mg |

By changing the ratio of the compound No.2 and lactose, capsules, which contains 10mg, 30mg, 50mg or 100mg of the compound No.2, were prepared.

3) Granule

| | |
|---|---|
| compound No.2 | 50mg |
| lactose | 54mg |
| crystalline cellulose | 20mg |
| polyvinylpyrrolidone K-30 | 5mg |
| magnesium stearate | 1mg |
| | 130mg |

Pharmacological Test

The rat liver disorder model caused by $CCl_4$ is generally used to examine the efficacy of a drug for liver diseases.

We examined the efficacy of the compound(s) of this invention on liver disorder using the rat model. Furthermore, we examined the immunomodulating effect of the compound(s) of this invention using immunoresponse against sheep red blood cells of mouse, which is generally used to examine the efficacy on immune system.

1) The effect on the liver disorder caused by $CCl_4$ .

The test compound was suspended in tragacanth gum solution and administered orally to male Wistar rats ( 5rats a group) at a dose of 300mg/kg.

Thirty minutes later, $CCl_4$ a liver disorder inducer, was given intraperitoneally at a dose of 0.25ml/kg.

Serum GOT and GPT levels were measured 24 hours after the administration of $CCl_4$ . To a control, 0.5% tragacanth gum solution was given. The results of the experiment with the compound No.2, a typical compound of this invention, is shown in the Table 1.

Table 1

| Test Compound | GOT | GPT |
|---|---|---|
| Control | 18693 | 10026 |
| Compound No.2 | 13605 | 6558 |

As shown in Table 1, the activity of serum transaminase of the group given the compound of the invention was significantly lower than that of the control. The result proved that the compound of this invention has an excellent effect on liver disorder.

2) The effect on immune response against sheep red blood cells of mouse.

According to the method of Iso ( Int. J. Immunotherapy, 1, 93 (1985)), 5 x $10^8$ sheep red blood cells were administered intraperitoneally to female BALB/c mice ( 3 to 5 mice a group ) and immunized.

After immunization, the test compound suspended in 1% methyl cellulose solution was administered continuously for 4 days.

Mice were killed and the number of haemolytic plaque-forming spleen cells were measured.

50% suppressive dose was calculated based on the cell count. For a comparison, the similar test with the known compound of the formula[VI] described in US Patent 4305958 was performed.

The results are shown in Table 2.

Table 2

| Test Compound | 50% suppressive dose |
|---|---|
| Compound No.2 | 19.1 mg/kg |
| known compound | 90.6 mg/kg |

As shown in Table 2, the compound of this invention shows excellent immunosuppressive effect and its effect is more potent than that of the known compound.

Toxicity Test

Acute Toxicity

The compound No.2 was suspended in 0.5% methyl cellulose solution at 20% concentration. The solution was administered orally to ddY mice ( male, 5 weeks age, 6 mice a group ) at a dose of 2000mg/kg.

Result

Toxicity of the compound No.2 was weak with a single case of death.

$LD_{50}$ was over 2000 mg/kg.

## Claims

1. A compound of the formula [I]

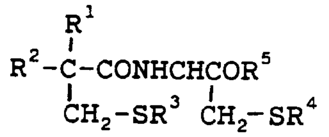

[I]

wherein

$R^1$ and $R^2$ are the same straight or branched chain $C_1$-$C_6$ alkyl;

$R^3$ and $R^4$ are the same or different and are selected from hydrogen, straight or branched chain $C_1$-$C_6$ alkyl, straight or branched chain $C_1$-$C_6$ alkanoyl, optionally substituted phenyl straight or branched chain $C_1$-$C_6$ alkyl and optionally substituted phenylcarbonyl, wherein the optional substituents are straight or branched chain $C_1$-$C_6$ alkyl, straight or branched chain $C_1$-$C_6$ alkoxy or halogen; and

$R^5$is hydroxy, straight or branched chain $C_1$-$C_6$ alkoxy, amino or straight or branched chain $C_1$-$C_6$ alkylamino;

or a pharmaceutically acceptable salt thereof.

2. N-(2,2-dimethyl-3-mercaptopropionyl)-L-cysteine.

3. A pharmaceutical composition useful for the treatment of liver disorder, comprising (i) a pharmaceutical carrier and (ii) a compound of the formula [I] as defined in claim 1, or a pharmaceutically acceptable salt thereof, in an amount sufficient for the treatment of liver disorder.

4. A pharmaceutical composition useful for the treatment of autoimmune diseases, comprising (i) a pharmaceutical carrier and (ii) a compound of the formula [I] as defined in claim 1, or a pharmaceutically acceptable salt thereof, in an amount sufficient for the treatment of autoimmune diseases.

5. A process for preparing a compound of the formula [I] as defined in claim 1 or a pharmaceutically acceptable salt thereof, which comprises reacting a compound of the formula [II] with a compound of the formula [III] or an active derivative thereof, or reacting a compound of the formula [II] with polythioester of the formula [IV];

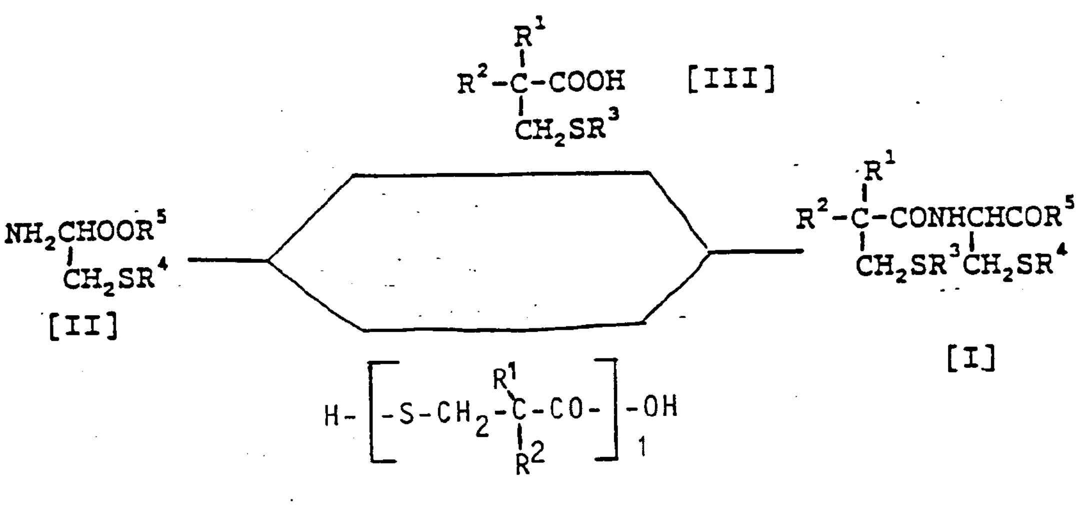

[III]

[II]

[I]

[IV]

wherein

$R^1$, $R^2$; $R^3$, $R^4$ and $R^5$ are as defined in claim 1;

1 is a polymerization degree having a mean molecular weight of 200 - 1500;

when $R^3$ and/or $R^4$ of the compound of the formula[I] are/is hydrogen, if necessary, the group(s)

can be converted into other groups than hydrogen after the above-mentioned reaction; when $R^5$ of the compound of the formula[I] is hydroxy, if necessary, the group can be converted into other groups than hydroxy after the above-mentioned reaction; and when $R^3$, $R^4$ and/or $R^5$ are used as a protective group, if necessary, the protective group can be removed.

**6.** A process as claimed in claim 5, wherein the compound of the formula[I] is N-(2,2-dimethyl-3-mercaptopropionyl)-L-cysteine.

## Patentansprüche

**1.** Eine Verbindung der Formel [I]

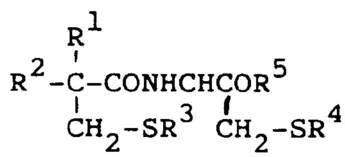

[I]

worin

$R^1$ und $R^2$ die gleichen gerad- oder verzweigtkettigen Alkylgruppen mit 1 bis 6 Kohlenstoffatomen;

$R^3$ und $R^4$ gleich oder verschieden und ausgewählt sind aus Wasserstoff, gerad- oder verzweigtkettigen Alkylgruppen mit 1 bis 6 Kohlenstoffatomen, gerad- oder verzweigtkettigen Alkanoylgruppen mit 1 bis 6 Kohlenstoffatomen, gegebenenfalls substituierten Phenyl - gerad-oder verzweigtkettig $C_1$-bis $C_6$-alkylgruppen und einer gegebenenfalls substituierten phenylcarbonylgruppe, wobei die gegebenenfalls vorhandenen Substituenten gerad- oder verzweigtkettige Alkyl-, gerad- oder verzweigtkettige Alkoxygruppen mit jeweils 1 bis 6 Kohlenstoffatomen oder Halogen sind; und

$R^5$ eine Hydroxy-, gerad- oder verzweigtkettige Alkoxygruppe mit 1 bis 6 Kohlenstoffatomen, eine Amino- oder gerad- oder verzeigtkettige $C_1$-bis $C_6$-Alkylaminogruppe ist; oder ein pharmazeutisch brauchbares Salz derselben.

**2.** N-(2,2-Dimethyl-3-mercaptopropionyl)-L-cystein.

**3.** Für die Behandlung von Lebererkrankungen brauchbare pharmazeutische Zusammensetzung mit einem Gehalt an (i) einem pharmazeutischen Träger und (ii) einer Verbindung der Formel [I], wie in Anspruch 1 definiert, oder einem pharmazeutisch brauchbarem Salz derselben in einer zur Behandlung der Lebererkrankung ausreichenden Menge.

**4.** Zur Behandlung von Autoimmunkrankheiten brauchbare pharmazeutische Zusammensetzung mit einem Gehalt an (i) einem pharmazeutischen Träger und (ii) einer Verbindung der Formel [I], wie in Anspruch 1 definiert, oder einem pharmazeutisch brauchbaren Salz derselben in einer zur Behandlung der Autoimmunkrankheiten ausreichenden Menge.

**5.** Verfahren zur Herstellung einer Verbindung der Formel [I], wie in Anspruch 1 definiert, oder eines pharmazeutisch brauchbaren Salzes derselben, welches die Umsetzung einer Verbindung der Formel [II] mit einer Verbindung der Formel [III] oder einem aktiven Derivat derselben, oder die Umsetzung einer Verbindung der Formel [II] mit einem Polythioester der Formel [IV] umfaßt,

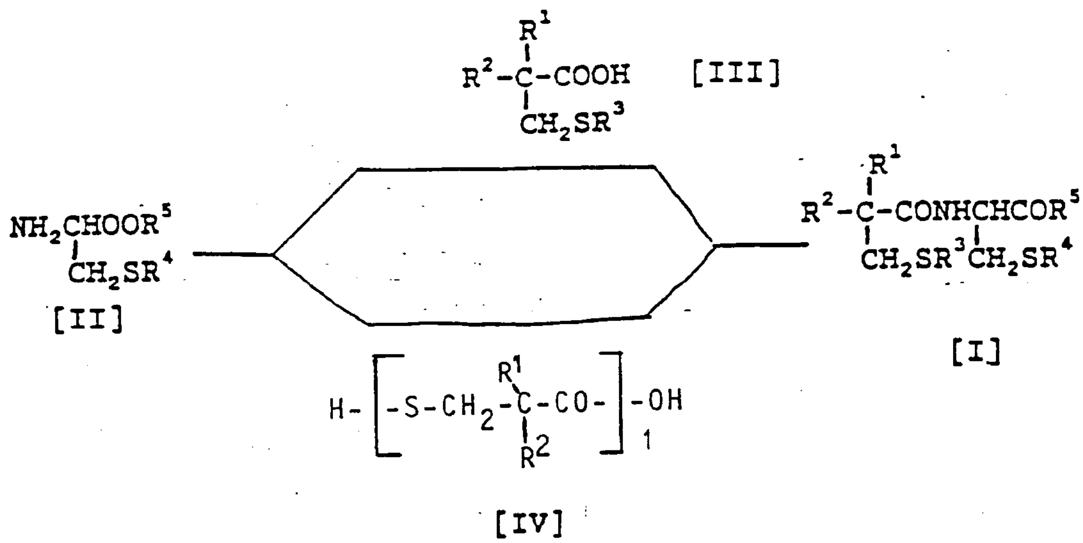

[III] [II] [I] [IV]

worin

$R^1$, $R^2$, $R^3$, $R^4$ und $R^5$ die in Anspruch 1 definierten Bedeutungen besitzen, und 1 einen Polymerisationsgrad mit einem mittleren Molekulargewicht von 200 bis 1500 bedeutet;

wobei, wenn $R^3$ und/oder $R^4$ der Verbindung der Formel [I] Wasserstoff ist bzw. sind, die Gruppe(n) erforderlichenfalls in andere Gruppen als Wasserstoff nach der zuvor genannten Umsetzung überführt werden kann bzw. können; wenn $R^5$ der Verbindung der Formel [I] eine Hydroxygruppe ist, diese erforderlichenfalls in andere Gruppen als die Hydroxygruppe nach der zuvor genannten Umsetzung überführt werden kann; und, wenn $R^3$, $R^4$ und/oder $R^5$ als Schutzgruppe benutzt werden, die Schutzgruppe erforderlichenfalls entfernt werden kann.

6. Verfahren gemäß Anspruch 5, wobei die Verbindung der Formel [I] N-(2,2-Dimethyl-3-mercaptopropionyl)-L-cystein ist.

**Revendications**

1. Un composé de la formule [I]

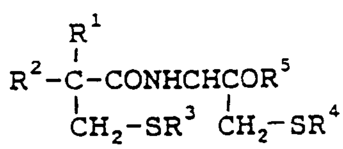

[I]

dans laquelle

$R^1$ et $R^2$ sont les mêmes groupements alkyles à chaîne droite ou ramifiée en $C_1$-$C_6$

$R^3$ et $R^4$ sont identiques ou différents et sont choisis parmi l'hydrogène, les groupements alkyles à chaîne droite ou ramifiée en $C_1$-$C_6$, les groupements alcanoyles à chaîne droite ou ramifiée en $C_1$-$C_6$, les groupements phénylalkyles à chaîne droite ou ramifiée en $C_1$-$C_6$ facultativement substitués et le groupement phénylcarbonyle facultativement substitué dans lesquels les substituants optionnels sont un groupement alkyle à chaîne droite ou ramifiée en $C_1$-$C_6$, un groupement alcoxy à chaîne droite ou ramifiée en $C_1$-$C_6$ ou un halogène et $R^5$ est un groupement hydroxy, un groupement alcoxy à chaîne droite ou ramifiée en $C_1$-$C_6$, un groupement amino ou un groupement alkylamino à chaîne droite ou ramifiée en $C_1$-$C_6$ ou un de ses sels pharmaceutiquement acceptable.

2. La N-(2,2-diméthyl-3-mercaptopropionyl)-L-cystéine.

3. Une composition pharmaceutique utile pour le traitement d'un trouble du foie, comprenant (i) un véhicule pharmaceutique et (ii) un composé de la formule [I] telle que définie dans la revendication 1, ou un de

ses sels pharmaceutiquement acceptables, en quantité suffisante pour le traitement du trouble du foie.

4. Une composition pharmaceutique utile pour le traitement de maladies autoimmunes comprenant (i) un véhicule pharmaceutique et (ii) un composé de la formule [I] telle que définie dans la revendication 1 ou un de ses sels pharmaceutiquement acceptables, en quantité suffisante pour le traitement des maladies autoimmunes.

5. Un procédé de préparation d'un composé de la formule [I] telle que définie dans la revendication 1 ou d'un de ses sels pharmaceutiquement acceptables, qui comprend la réaction d'un composé de la formule [II] avec un composé de la formule [III] ou un de ses dérivés actifs ou la réaction d'un composé de la formule [II] avec un polythioester de la formule [IV]

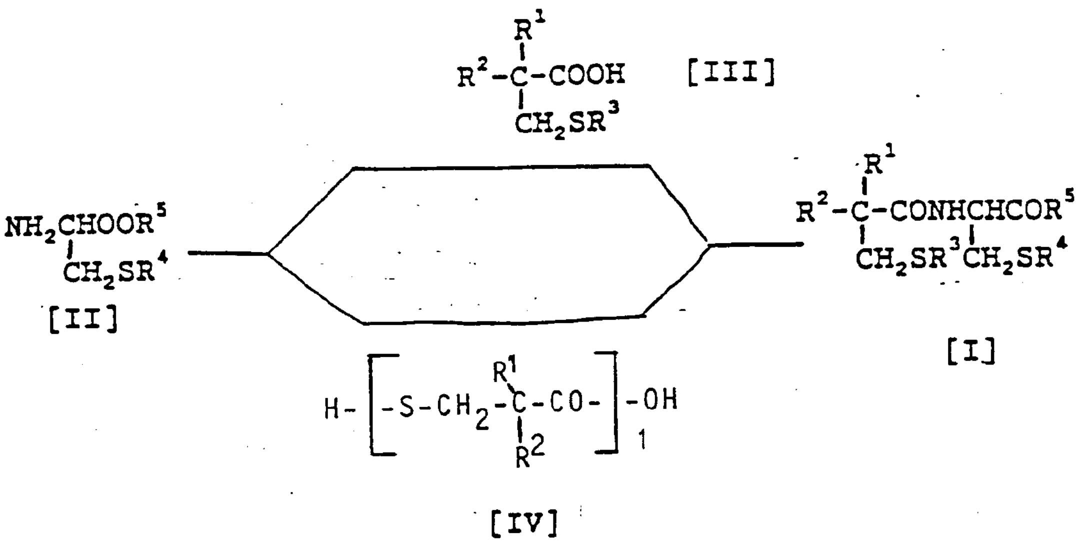

[III] [II] [I] [IV]

dans laquelle $R^1$, $R^2$, $R^3$, $R^4$ et $R^5$ sont tels que définis dans la revendication 1;
1 est un degré de polymérisation ayant un poids moléculaire moyen de 200-1500;
lorsque $R^3$ et/ou $R^4$ du composé de la formule [I] sont/est de l'hydrogène, si nécessaire, le (les) groupement(s) peut (peuvent) être transformé(s) en d'autres groupements que l'hydrogène après la réaction mentionnée ci-avant; lorsque $R^5$ du composé de la formule [I] est un groupement hydroxy, si nécessaire, le groupement peut être transformé en d'autres groupements que le groupement hydroxy après la réaction mentionnée ci-avant et, lorsque $R^3$, $R^4$ et/ou $R^5$ sont utilisés comme groupement protecteur, si nécessaire, le groupement protecteur peut être enlevé.

6. Un procédé tel que revendiqué dans la revendication 5, dans lequel le composé de la formule [I] est la N-(2,2-diméthyl-3-mercaptopropionyl)-L-cystéine.